# EUROPÄISCHE PATENTSCHRIFT

(11) **EP 0 574 667 B1**
(45) Veröffentlichungstag und Bekanntmachung des Hinweises auf die Patenterteilung: **04.06.1997**
(21) Anmeldenummer: 93106040.4
(22) Anmeldetag: 14.04.1993
(51) Int. Cl.: C07D 211/94

(54) **Verfahren zur Herstellung von 2,2,6,6-Tetramethylpiperidin-N-oxyl und in seiner 4-Stellung substituierten Derivaten**
Process for the preparation of 2,2,6,6-tetramethylpiperidin-N-oxyls, and their 4-substituted derivatives
Procédé pour la préparation des dérivés 4-substitués de 2,2,6,6-tétraméthylpipéridine-N-oxyls

(30) Priorität: 13.06.1992 DE 4219459
(43) Veröffentlichungstag der Anmeldung: 22.12.1993
(73) Patentinhaber: HÜLS AKTIENGESELLSCHAFT, 45764 Marl (DE)
(72) Erfinder: Büschken, Wilfried, Dr., W-4358 Haltern 6 (DE); Kaufhold, Manfred, Dr., W-4370 Marl (DE); Bickert, Peter, Dr., W-4400 Münster (DE)

(56) Entgegenhaltungen:
- EP-A- 0 157 738
- EP-A- 0 233 622
- EP-A- 0 488 403
- GB-A- 983 134
- GB-A- 2 048 842
- SOVIET PATENTS ABSTRACTS Section Ch, Week 9124, 31. Juli 1991 Derwent Publications Ltd., London, GB; Class C, AN 91-176790 & SU-A-1583415 (POPOVA EG) 7 August 1990
- SYNTHESIS Nr. 11, November 1987, STUTTGART Seiten 1015 - 1017 PAUL BROUGHAM ET AL. 'Oxidation reactions using Magnesium Monoperphthalate.....'

## Beschreibung

Die Erfindung betrifft ein Verfahren zur Herstellung von 2,2,6,6-Tetramethylpiperidin-N-oxyl (im folgenden mit TEMP-N-oxyl abgekürzt) und seinen in 4-Stellung substituierten Derivaten (der Formel 1) durch Oxidation von 2,2,6,6-Tetramethylpiperidin (im folgenden mit TEMP abgekürzt) bzw. seinen in 4-Stellung substituierten Derivaten (der Formel 2) mit Wasserstoffsuperoxid in Gegenwart von wasserlöslichen Leicht- oder Schwermetallsalzen als Katalysatoren in sehr geringen Konzentrationen. 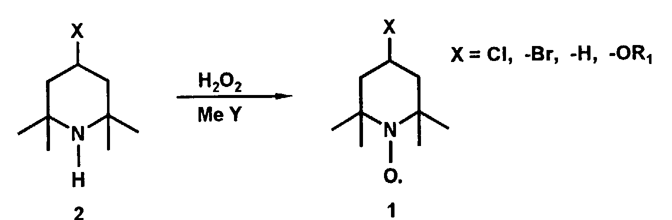 R₁ = H, -CH₃, -C₂H₅, n-, i-C₃H₇, i-, n-, tert.-C₄-Alkyl, 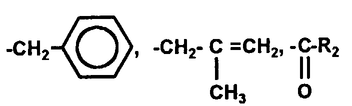 R₂ = H, -CH₃, -C₂H₅, -C₃H₇, i-, n-, tert.-C₄-Alkyl,

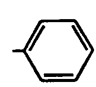

Synthesen von N-Oxylen durch Oxidation von den entsprechenden tertiären Aminen sind aus der Literatur bekannt, sie unterscheiden sich vor allem hinsichtlich des eingesetzten Oxidationsmittels: So führen R. Winter und R. Malherbe die Oxidation mit organischen Hydroperoxiden, z. B. tert.-Butylhydroperoxid, durch (s. EP-A-O 157 738); sie verwenden also ein teures Oxidationsmittel und erhalten in mindestens stöchiometrischen Mengen ein Kopplungsprodukt. Dieses Problem besteht auch bei den häufig als Oxidationsmittel empfohlenen Percarbonsäuren.

Chou et al. verwenden beispielsweise 3-Chlorperoxidbenzoesäure als Oxidationsmittel (s. J. Org. Chem. 39 [1947] 2356, 2360).

Wesentlich günstiger ist dagegen die Verwendung von Wasserstoffsuperoxid als Oxidationsmittel, weil es preiswert ist und als Kopplungsprodukt Wasser entsteht. D. P. Young und Rozantsev et al. empfehlen, Wasserstoffsuperoxid als Oxidationsmittel und ein Salz der Wolframsäure als Katalysator (s. GB-P 1 199 351 und Tetrahedron 20 [1964] 131, 137). Nachteilig bei diesem Verfahren sind die extrem langen Reaktionszeiten von mehreren Tagen und das Problem der Entsorgung des Katalysators, da dieser aus Umweltschutzgründen nicht in das Abwasser gelangen darf. Auch bei der Verwendung von Natriumcarbonat muß man zu lange Reaktionszeiten in Kauf nehmen (s. Soviet physics Doklaay 261,1,103-110, [1981]).
Durch Einsatz von Phosphorwolframsäure wird zwar die Reaktionszeit erheblich verkürzt, das Problem der Entsorgung des Katalysators und seine aufwendige Herstellung bleiben aber bestehen (s. Cf. R. Briere, H. Lemaire und A. Rassat, Bull. Soc. Chim. France, 11, 3273 [1965]).

In EP-A-0 488 403 werden zur Oxidation von sterisch gehinderten Aminen speziell hergestellte Titan enthaltende Katalysatoren in großen Mengen verwendet. Der Katalysator muß nach der Reaktion abgetrennt werden.

Nach SU-A-1 583 415 wird als Katalysator das Natriumsalz der Phosphorwolframsäure verwendet. Dieser Katalysator wird separat hergestellt und muß nach der Reaktion abgetrennt werden.

EP-A-0 233 622 betrifft durch Zink und Cadmium katalysierte Oxidationen von sekundären Aminen zu Hydroxylaminen.

GB-A-983 134 beschreibt die Oxidation eines sekundären Amins mit einem Acylperoxid zu dem entsprechenden Acylhydroxylamin.

In GB-A-2 048 842 wird die Herstellung von festen, wasserfreien anorganischen Peroxiden beschrieben. Dabei werden Metalloxide mit H₂O₂ in konzentrierten Lösungen umgesetzt und direkt getrocknet. Dabei können anorganische Peroxide entstehen.

In Synthesis, 1987, 1015-17, wird gezeigt, daß das Oxidationsmittel Magnesiummonoperphthalat bessere Eigenschaften als m-Chlorperoxybenzoesäure besitzt. Beide Oxidationsmittel sind jedoch im Vergleich zu H₂O₂ teuer und hinsichtlich der Abfallbeseitigung problematisch.

Alle bekannten Oxidationsverfahren haben einen hohen Verbrauch an Chemikalien, sind technisch aufwendig und einige sehr zeitraubend und alle Verfahren führen zu Problemen der Abfallbeseitigung.

Aufgabe der Erfindung war es daher, ein Verfahren zu entwickeln, bei dem TEMP und seine in 4-Stellung substituierten Derivate mit Wasserstoffsuperoxid oxidiert werden können, ohne daß der dazu benötigte Katalysator entsorgt werden muß.

Es besteht ein großes Interesse an einem derartigen Verfahren, weil das N-Oxyl des TEMP und seiner Derivate wichtige Stabilisatoren für polymere Werkstoffe sind und als Lichtschutzmittel verwendet werden. Außerdem finden diese N-Oxyle als Redox-Katalysatoren zunehmend an Bedeutung. Darüber hinaus dienen sie als Vorstufen für weitere N-Oxylderivate.

Gelöst wurde diese Aufgabe dadurch, daß zur Katalyse Erdalkalimetallsalze eingesetzt werden, wobei das molare Verhältnis von 2,2,6,6-Tetramethylpiperidin und seinen in 4-Stellung substituierten Derivaten zu den Metallsalzen 10⁵ : 1 bis 10 : 1 beträgt.

Gegenstand der Erfindung ist daher ein Verfahren zur Herstellung von 2,2,6,6-Tetramethylpiperidin-N-oxyl und seinen in 4-Stellung substituierten Derivaten der Formel 1 durch Oxidation von 2,2,6,6-Tetramethylpiperidin und seinen in 4-Stellung substituierten Derivaten der Formel 2 mittels Wasserstoffsuperoxid. 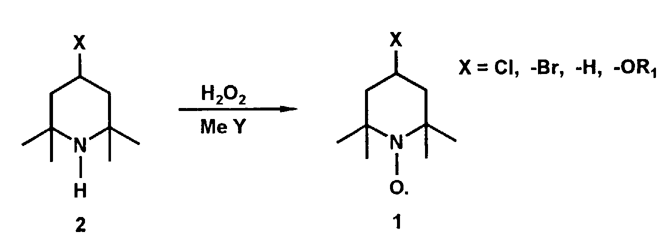 R₁ = H, -CH₃, -C₂H₅, n-, i-C₃H₇, i-, n-, tert.-C₄-Alkyl, 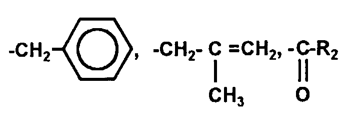 R₂ = H, -CH₃, -C₂H₅, -C₃H₇, i-, n-, tert.-C₄-Alkyl, 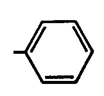 dadurch gekennzeichnet, daß die Oxidation unter der Katalyse von Erdalkalimetallsalzen erfolgt.

Überraschenderweise wurde gefunden, daß TEMP und seine Derivate durch Wasserstoffsuperoxid unter der Katalyse von Erdalkalimetallsalzen nahezu quantitativ mit sehr hoher Selektivität von über 90 % in einigen Fällen sogar über 97 % oxidiert werden, wobei die Konzentrationen des Metallsalzes extrem niedrig sind. Als Erdalkalimetallsalze können Verbindungen des Magnesiums, Calciums, Bariums und Strontiums eingesetzt werden, z. B. in Form der Chloride, Sulfate, Nitrate, Phosphate, Hydroxide usw. Voraussetzung ist, daß die Salze wasserlöslich sind und in dem Reaktionsgemisch in Ionenform vorliegen. Selbstverständlich können auch alle Verbindungen eingesetzt werden, die mit dem Reaktionsgemisch reagieren und dann in Ionen dissoziieren, wie z. B. Metallalkoholate oder Grignard-Verbindungen oder die Metalle selbst. Die Salze können als reiner Stoff, in Form einer Lösung und auch als Gemische eingesetzt werden.

Entscheidend und sehr überraschend ist die extrem niedrige Konzentration, die völlig ausreicht, um die guten Ausbeuten zu erzielen:

Das molare Verhältnis von Einsatzstoff zum Metallsalz beträgt 10⁵ : 1 bis 10 : 1, vorzugsweise 10⁵ : 1 bis 10² : 1, insbesondere 10⁴ : 1. Wegen dieser geringen Konzentration ist der Katalysatorverbrauch sehr gering. Außerdem ist es vorteilhaft, daß es äußerst billige Chemikalien sind. Ein weiterer Vorteil ist, daß es sich um Chemikalien handelt, die die Umwelt nicht belasten, da Verbindungen, z. B. des Magnesiums und Calciums in der Natur weit verbreitet sind, so daß Spuren dieser Verbindungen in Abfallprodukten nicht zu Umweltschutzproblemen führen können.

Die Umsetzung wird in wäßriger Lösung oder in Suspension durchgeführt. Ob ein Lösungsmittel zusätzlich eingesetzt wird oder nicht, richtet sich nach der Polarität bzw. nach der Löslichkeit der zu oxidierenden Verbindung in Wasser. Wird z. B. 4-Hydroxi-TEMP (X = OR₁, R₁ = H), eine sehr polare Verbindung eingesetzt, so erübrigt sich jedes weitere Lösungsmittel.

Umgekehrt setzt man bei dem sehr unpolaren TEMP selbst (X = H) ein Lösungsmittel zu, das die Löslichkeit von TEMP in Wasser erhöht. Als Lösungsmittel werden aus wirtschaftlichen Gründen die einfachen niedrig siedenden Alkohole oder Diole wie Methanol, Ethanol, n- oder iso-Propanol, tert.-, iso- oder n-Butanole, Ethylenglykol, Propylenglykol, Ethylendiglykol, Propylendiglykol usw., Alkylglykolether, Dioxan-1.4 oder -1.3, Tetrahydrofuran und ähnliche Verbindungen eingesetzt. Bevorzugte Lösungsmittel sind aus Kostengründen Methanol, Ethanol und iso-Propanol. Anstelle eines reinen Lösungsmittels können auch Gemische von zwei oder mehr Lösungsmitteln eingesetzt werden. Die Gewichtsmenge an Lösungsmittel beträgt das 1- bis 10 fache, vorzugsweise das 2- bis 1,lfache der Gewichtsmenge an TEMP bzw. an TEMP-Derivat. Die günstigste Menge bei der jeweils einzusetzenden Verbindung kann nötigenfalls in einem Vorversuch ermittelt werden. Je polarer die Verbindung ist, umso geringer kann die Lösungsmittelmenge gewählt werden, TEMP und 4-Hydroxi-TEMP sind als zwei Extreme anzusehen, zwischen denen alle übrigen Derivate liegen.

Wasserstoffsuperoxid kann in den handelsüblichen Formen als 10- bis 90%ige wäßrige Lösung eingesetzt werden.

Die praktische Durchführung erfolgt beispielsweise in folgender Weise:

TEMP bzw. ein TEMP-Derivat, Katalysator und Wasser werden vorgelegt, wobei die Gewichtsmenge an Wasser das 2- bis 0,lfache, vorzugweise das 0,8- bis 0,3fache der Gewichtsmenge an TEMP bzw. TEMP-Derivat entspricht. Die Temperatur des Reaktionsgemisches wird auf 0 bis 100 °C, bevorzugt auf 40 bis 90 °C, eingestellt. Innerhalb von 0,1 bis 2 Stunden wird Wasserstoffsuperoxid zugetropft und anschließend bei der gewählten Temperatur noch 1 bis 30, vorzugsweise 5 bis 15 Stunden, weitergerührt. Das Molverhältnis TEMP bzw. TEMP-Derivat zu Wasserstoffsuperoxid beträgt 1 : 1 bis 1 : 10, vorzugsweise 1 : 1 bis 1 : 5, insbesondere 1 : 1,5 bis 1 : 2,5.
Besonders vorteilhaft wird H₂O₂ in 105 bis 135 % der stöchiometrischen Menge eingesetzt.

Der Reaktionsverlauf wird mittels gaschromatographischer Analyse überwacht.

Die Aufarbeitung erfolgt nach den üblichen Methoden und ist in den meisten Fällen sehr einfach, da die Ausgangsverbindung fast vollständig umgesetzt wird. Beispielsweise wird bei der Oxidation des 4-Hydroxi-TEMP, bei der kein Lösungsmittel benötigt wird, einfach so aufgearbeitet, daß Wasser im Vakuum abdestilliert wird. Als Rückstand verbleibt das entsprechende N-Oxyl mit einer Reinheit von über 99 %. Da die Katalysatorkonzentration im Endprodukt kleiner als 100 ppm beträgt, erübrigt sich für die meisten Anwendungszwecke eine Abtrennung. Selbstverständlich kann das Reaktionsprodukt beispielsweise durch Umkristallisieren gereinigt und dabei der Katalysator entfernt werden.

Die folgenden Beispiele sollen das erfindungsgemäße Verfahren näher erläutern, ohne seine Anwendungsgebiete einzugrenzen.

### Beispiel 1

### Oxidation von 4-Hydroxi-TEMP

Man benutzt eine Glasapparatur, die aus einem Dreihalskolben mit Rührer, Thermometer und Tropftrichter besteht.

### Man setzt ein

| | |
|---|---|
| 314 g (2,0 Mol) | 4-Hydroxi-TEMP |
| 250 ml | Wasser |
| 11,6 mg (0,2 mMol) | Magnesiumhydroxid |
| 453 g (4,0 Mol) | Wasserstoffsuperoxid (30%ig) |

Durch Zugabe von 4-Hydroxi-TEMP zum Wasser wird eine Suspension hergestellt, das Magnesiumhydroxid zugegeben und auf 70 °C erwärmt. Bei dieser Temperatur wird während 2 Stunden die 30 %ige wäßrige H₂O₂-Lösung zugetropft. Es wird weitere 10 Stunden bei 70 °C gerührt. Bei 90 °C und einem Druck von 40 hPa wird Wasser abgezogen. Der Rückstand (340 g) erstarrt beim Abkühlen auf Raumtemperatur. Der Gehalt an 4-Hydroxi-TEMP-N-oxyl ist 99,8 %. Dies entspricht einer Ausbeute von 98,6 % der Theorie. Der Schmelzpunkt liegt bei 71 bis 72 °C.

### Beispiel 2

### Oxidation von 4-Hydroxi-TEMP

Man benutzt die im Beispiel 1 beschriebene Apparatur, setzt die hier genannten Mengen an 4-Hydroxi-TEMP und Wasser ein und setzt nun als Katalysator

| | |
|---|---|
| 40,6 mg (0,2 mMol) | Magnesiumchlorid mit 6 Kristallwasser, MgCl₂ · 6 H₂O zu. |

Dann wird bei 70 °C innerhalb von 2 Stunden 453 g (4 Mol) 30%ige wäßrige H₂O₂-Lösung zugetropft. Es wird 15 Stunden bei 70 °C nachgerührt. Nach dem Abziehen des Wassers bleibt 342 g Rückstand mit einem TAA-ol-oxyl-Gehalt von 99,3 %. Dies entspricht einer Ausbeute von 98,7 %. Der Schmelzpunkt liegt bei 71 bis 72 °C.

### Beipsiel 3

### Oxidation von 4-Hydroxi-TEMP

Man benutzt die im Beispiel 1 beschriebene Apparatur, setzt die hier genannten Mengen an 4-Hydroxi-TEMP und Wasser ein und setzt als Katalysator

| | |
|---|---|
| 114 mg (0,5 mMol) | Magnesiumsulfat mit 6 Kristallwasser, MgSO₄ · 6 H₂O zu. |

Dann wird bei 70 °C innerhalb von 2 Stunden 453 g (4,0 Mol) 30%ige wäßrige H₂O₂-Lösung zugetropft. Es wird 15 Stunden bei 70 °C nachgerührt. Nach dem Abziehen des Wassers bleibt 340 g Rückstand mit einem TAA-ol-N-oxyl-Gehalt von 99,2 %. Dies entspricht einer Ausbeute von 98 % der Theorie. Der Schmelzpunkt liegt bei 71 bis 72 °C.

### Beispiel 4

### Oxidation von TEMP

Man benutzt die im Beispiel 1 beschriebene Apparatur, baut zusätzlich einen Rückflußkühler ein und setzt ein:

| | |
|---|---|
| 28,25 g (0,2 Mol) | TEMP |
| 45 ml | Methanol |
| 4,07 mg (0,02 mMol) | Magnesiumchlorid mit 6 Kristallwasser, MgCl₂ · 6 H₂O, gelöst in 1 ml Wasser. |

TEMP, Methanol und die Magnesiumchloridlösung werden zusammengegeben und auf 65 °C erwärmt. Dann werden innerhalb von 45 Minuten

| | |
|---|---|
| 45,3 g (0,4 Mol) | 30%ige H₂O₂-Lösung |

zugetropft und danach 7 Stunden lang bei 65 °C gerührt. Die gaschromatographische Analyse weist dann aus:

| | |
|---|---|
| Gehalt an TEMP | 4,4 % |
| Gehalt an TEMP-N-Oxyl | 92,5 % |

Der Umsatz lag also über 95 %.

Zur Reinigung des N-Oxyls werden 5 ml 10%ige Schwefelsäure zugegeben und das Reaktionsgemisch viermal mit 50 ml Cyclohexan extrahiert. Das Cyclohexan wird abdestilliert und 27,1 g N-Oxyl als dunkelrote Kristalle mit einer Reinheit von 98,7 % erhalten. Die Ausbeute beträgt 85,5 der Theorie, bezogen auf Einsatz.

### Beispiele 5 bis 10

Man benutzt die im Beispiel 1 beschriebene Apparatur und setzt die im Beispiel 4 genannten Produkte ein mit dem Unterschied, daß man anstelle des TEMP die in der folgenden Tabelle aufgeführten TEMP-Derivate verwendet. Oxidation und Aufarbeitung erfolgen wie im Beispiel 4 beschrieben. Man erhält die in der Tabelle angegebenen Ergebnisse.

### Zur Bezeichnung der Einsatzprodukte

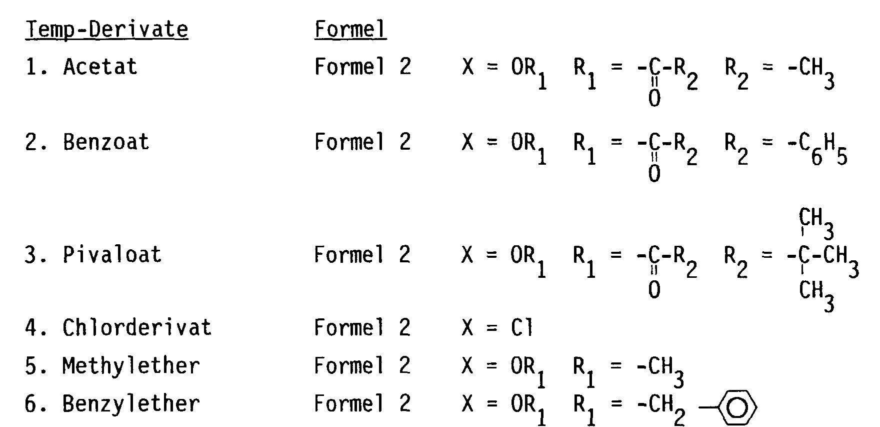

Die Endprodukte 1 bis 6 haben die mit Formel 1 beschriebene Struktur, wobei die Bedeutung von X, R₁ und R₂ gegenüber dem Einsatzprodukt unverändert bleibt.

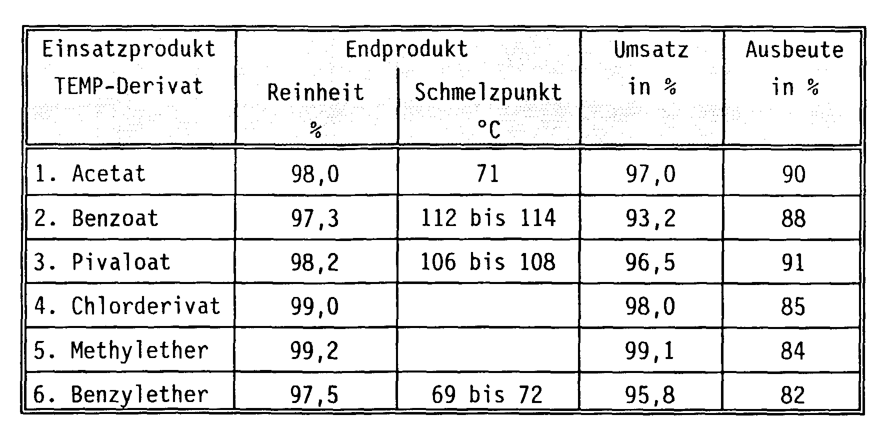

## Patentansprüche

1. Verfahren zur Herstellung von 2,2,6,6-Tetramethylpiperidin-N-oxyl und seinen in 4-Stellung substituierten Derivaten der Formel 1 durch Oxidation von 2,2,6,6-Tetramethylpiperidin und seinen in 4-Stellung subtituierten Derivaten der Formel 2 mittels Wasserstoffsuperoxid 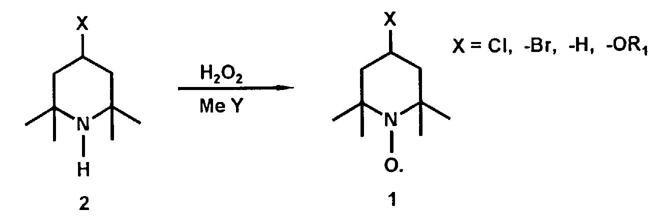 R₁ = H, -C₃, -C₂H₅, n-, i-C₃H₇, i-, n-, tert.-C₄-Alkyl, 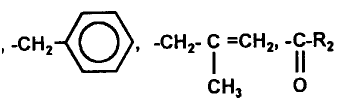 R₂ = H, -CH₃ -C₂H₅, -C₃H₇, i-, n-, tert.-C₄-Alkyl, 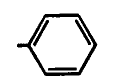 dadurch gekennzeichnet,
daß die Oxidation unter der Katalyse von Erdalkalimetallsalzen erfolgt, wobei das molare Verhältnis von 2,2,6,6-Tetramethylpiperidin und seinen in 4-Stellung substituierten Derivaten zu den Metallsalzen 10⁵ : 1 bis 10 : 1 beträgt.

2. Verfahren nach Anspruch 1,
dadurch gekennzeichnet,
daß die Erdalkalimetallsalze in Form ihrer Chloride, Sulfate, Nitrate, Phosphate und Hydroxide eingesetzt werden.

3. Verfahren nach den Ansprüchen 1 und 2,
dadurch gekennzeichnet,
daß als Katalysator Mg(OH)₂ und Magnesiumsalze sowie Verbindungen, die in wäßriger Lösung Magnesiumionen bilden, eingesetzt werden, insbesondere MgSO₄ · 6 H₂O, MgCl₂ · 6 H₂O und Mg(NO₃)₂.

4. Verfahren nach Anspruch 1,
dadurch gekennzeichnet,
daß das molare Verhältnis zwischen 2,2,6,6-Tetramethylpiperidin und seinen Derivaten zu den Salzen zwischen 10⁵ : 1 bis 10 : 1, vorzugsweise 10⁵ : 1 bis 10²: 1 und besonders bevorzugt 10⁴ : 1 beträgt.

5. Verfahren nach Anspruch 1,
dadurch gekennzeichnet,
daß die Umsetzung in homogener wäßriger Lösung oder in wäßriger Suspension erfolgt.

6. Verfahren nach Anspruch 1,
dadurch gekennzeichnet,
daß die Umsetzung unter Zusatz eines Lösungsmittels, insbesonders niedrig siedender Alkohole oder Diole, erfolgt.

7. Verfahren nach Anspruch 6,
dadurch gekennzeichnet,
daß die Umsetzung unter Zusatz von Methanol, Ethanol, n- oder iso-Propanol, tert.-, iso- oder n-Butanol, Ethylenglykol, Propylenglykol, Ethylendiglykol, Propylendiglykol, Alkylglykolether, insbesondere Methoxiethanol und Ethoxiethanol, Dioxan-1,3 oder -1,4 und Tetrahydrofuran erfolgt.

8. Verfahren nach den Ansprüchen 1 und 7,
dadurch gekennzeichnet,
daß H₂O₂ in einem Konzentrationsbereich von 10 bis 90 %, insbesondere von 30 bis 40 %, eingesetzt wird.

9. Verfahren nach den Ansprüchen 1 bis 8,
dadurch gekennzeichnet,
daß H₂O₂ in 105 bis 135 % der stöchiometrischen Menge eingesetzt wird.

10. Verfahren nach den Ansprüchen 1 bis 9 ,
dadurch gekennzeichnet,
daß die Umsetzung im Temperaturbereich 0 bis 100 °C, insbesondere 40 bis 90 °C, durchgeführt wird.

## Claims

1. A process for preparing 2,2,6,6-tetramethylpiperidin-N-oxyl and its 4-substituted derivatives of the formula 1 by oxidation of 2,2,6,6-tetramethylpiperidine and its 4-substituted derivatives of the formula 2 by means of hydrogen superoxide, 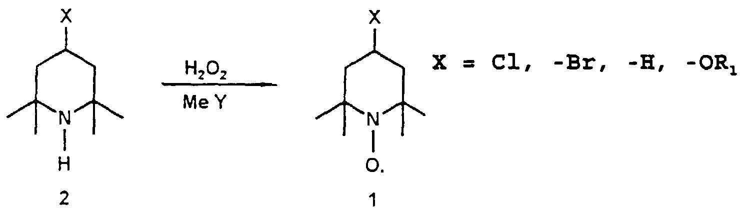 R₁ = H, -CH₃, -C₂H₅, n-, i-C₃H₇, i-, n-, tert-C₄-alkyl, 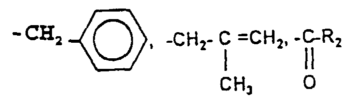 R₂ = H, -CH₃, -C₂H₅, -C₃H₇, i-, n-, tert-C₄-alkyl, 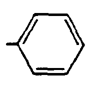 characterized in that the oxidation is carried out in the presence of alkaline earth metal salts as catalysts, where the molar ratio of 2,2,6,6-tetramethylpiperidine and its 4-substituted derivatives to the metal salts is from 10⁵ : 1 to 10 : 1.

2. A process according to claim 1, characterized in that the alkaline earth metal salts are used in the form of their chlorides, sulphates, nitrates, phosphates and hydroxides.

3. A process according to either of claims 1 and 2, characterized in that Mg(OH)₂, magnesium salts and compounds which form magnesium ions in aqueous solution are used as catalyst, in particular MgSO₄ · 6 H₂O, MgCl₂ · 6 H₂O and Mg(NO₃)₂.

4. A process according to claim 1, characterized in that the molar ratio of 2,2,6,6-tetramethylpiperidine and its derivatives to the salts is from 10⁵ : 1 to 10 : 1, preferably from 10⁵ : 1 to 10² : 1 and particularly preferably 10⁴ : 1.

5. A process according to claim 1, characterized in that the reaction is carried out in homogeneous aqueous solution or in aqueous suspension.

6. A process according to claim 1, characterized in that the reaction is carried out with addition of a solvent, in particular low-boiling alcohols or diols.

7. A process according to claim 6, characterized in that the reaction is carried out with addition of methanol, ethanol, n- or iso-propanol, tert-, iso- or n-butanol, ethylene glycol, propylene glycol, ethylene diglycol, propylene diglycol, alkyl glycol ethers, in particular methoxyethanol and ethoxyethanol, 1,3- or 1,4-dioxane and tetrahydrofuran.

8. A process according to any of claims 1 to 7, characterized in that H₂O₂ is used in a concentration range from 10 to 90 %, in particular from 30 to 40 %.

9. A process according to any of claims 1 to 8, characterized in that H₂O₂ is used in an amount of from 105 to 135 % of the stoichiometric amount.

10. A process according to any of claims 1 to 9, characterized in that the reaction is carried out in the temperature range from 0 to 100°C, in particular from 40 to 90°C.

## Revendications

1. Procédé de fabrication de 2,2,6,6-tétraméthylpiperidine-N-Oxyl et de ses dérivés substitués en position 4 de formule I par oxydation de 2,2,6,6-tétraméthylpipéridine et de ses dérivés substitués en position 4 de formule 2, à l'aide de superoxyde d'hydrogène. 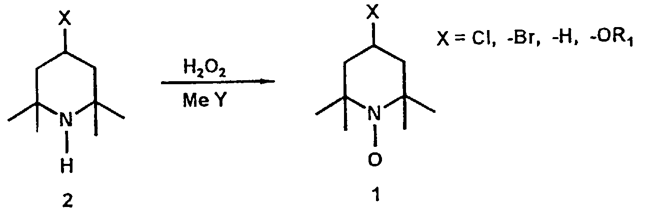 R₁ = H, -C₃, -C₂H₅, n-, i-C₃H₇, i-, n-, tert.-C₄-Alkyl, 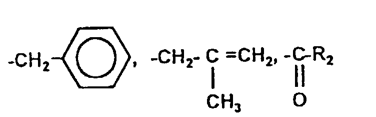 R₂ = H, -CH₃, -C₂H₅, -C₃H₇, i-, n-, tert-C₄-Alkyl 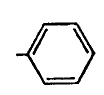 caractérisé en ce que
l'on effectue l'oxydation sous catalyse par des sels de métal alcalino-terreux, dans laquelle le rapport molaire de 2,2,6,6-tétraméthylpipéridine et de ses dérivés substitués en position 4, aux sels métalliques s'élève à 10⁵:1 à 10:1.

2. Procédé selon la revendication 1,
caractérisé en ce que
les sels de métal alcalino-terreux sont mis en oeuvre sous forme de leur chlorure, de leur sulfate, de leur nitrate, de leur phosphate et de leur hydroxyde.

3. Procédé selon la revendication 1 et 2,
caractérisé en ce que
l'on met en oeuvre comme catalyseur Mg(OH)₂ et des sels de magnésium ainsi que des composés qui forment en solution aqueuse des ions magnésium, en particulier SO4Mg 6 H₂O, Cl₂Mg 6 H₂O et (NO₃)₂Mg.

4. Procédé selon la revendication 1,
caractérisé en ce que
le rapport molaire entre la 2,2,6,6-tétraméthylpiperidine et ses dérivés aux sels, s'élève à 10⁵:1 à 10:1, de préférence 10⁵:1 à 10²:1 et d'une manière particulièrement préférée à 10⁴:1.

5. Procédé selon la revendication 1,
caractérisé en ce que
la réaction s'effectue en solution aqueuse homogène ou en suspension aqueuse.

6. Procédé selon la revendication 1,
caractérisé en ce que
la réaction s'effectue en ajoutant un solvant, en particulier des alcools à bas point d'ébullition ou des diols.

7. Procédé selon la revendication 6,
caractérisé en ce que
la réaction s'effectue en ajoutant du méthanol, de l'éthanol, du n-ou de l'isopropanol, du ter-, de l'iso- ou du n-butanol, de l'éthylèneglycol, du propylèneglycol, de l'éthylènediglycol, un éther d'alcoyle glycol, en particulier du méthoxyéthanol, et de l'éthoxyéthanol, du dioxanne-1,3 ou 1,4 et du tétrahydrofuranne.

8. Procédé selon les revendications 1 et 7,
caractérisé en ce que
l'on met en oeuvre H₂O₂ dans une plage de concentration de 10 à 90 %, en particulier de 30 à 40 %.

9. Procédé selon les revendications 1 à 8,
caractérisé en ce que
l'on met en oeuvre H₂O₂ en 105 à 135 % de la quantité stoechiométrique.

10. Procédé selon les revendications 1 à 9,
caractérisé en ce que
l'on exécute la réaction dans la plage de température allant de 0 à 100°C, en particulier de 40 à 90°C.
